# EUROPÄISCHE PATENTANMELDUNG

(11) **EP 1 234 553 A1**
(43) Veröffentlichungstag der Anmeldung: **28.08.2002**
(21) Anmeldenummer: 01103420.4
(22) Anmeldetag: 14.02.2001
(51) Int. Cl.: A61F 2/02, C12M 3/06, A01N 1/02

(54) **Bioartifizielles Gerät**

(71) Anmelder: Hochleitner Boris-Wolfgang, 6020 Innsbruck (AT); Margreiter, Raimund, 6103 Seefeld (AT)
(72) Erfinder: Hochleitner, Boris-Wolfgang, 6020 Innsbruck (AT)
(74) Vertreter: Torggler, Paul Norbert, Dr.

(57) **Zusammenfassung**

Ein bioartifizielles Gerät weist ein Reaktorgefäß (6) auf, in dem eine Zellkulturkammer (14) und eine Durchflußkammer (17, 21) für ein Nährmedium durch eine semipermeable Wandung getrennt sind. Wenn das bioartifizielle Gerät eine Leberfunktion erfüllen soll, ist auch eine Durchflußkammer (21) für Plasma und Blut vorgesehen. Jede Durchflußkammer (17, 21) ist durch einen schraubenförmig gewickelten Schlauch (18, 22) aus einem semipermeablen Material gebildet, der in der Zellkulturkammer (14) angeordnet ist.

## Beschreibung

Die Erfindung betrifft ein bioartifizielles Gerät mit einem Reaktorgefäß, in dem eine Zellkulturkammer und eine Durchflußkammer für ein Nährmedium durch eine semipermeable Wandung getrennt sind, und insbesondere ein bioartifizielles Gerät mit Leberfunktion, das mit einer Durchflußkammer für ein Nährmedium und mit einer Durchflußkammer für Plasma oder Blut versehen ist.

Derartige Geräte mit Leberfunktion sollen in der Lage sein, vorübergehend die Leberfunktion eines Patienten zu übernehmen, insbesondere im Falle einer der folgenden Erkrankungen:
1. Akutes Leberversagen (fulminanter Verlauf infektiöser Hepatitiden, Intoxikationen wie Knollenblätterpilzvergiftung, etc.)
2. Primäre Nichtfunktion eines Lebertransplantats
3. Überbrückung bis zur Lebertransplantation bei plötzlicher Verschlechterung einer chronischen Leber-Erkrankung
4. Intermittierende Behandlung bei chronischer Leberinsuffizienz.

Das akute Leberversagen ist eine Konstellation von klinischen Symptomen, die aufgrund eines plötzlichen Leberausfalls resultieren (Hoofnagle). Definitionsgemäß müssen hepatische Enzephalopathie, Koagulopathie und Ikterus vorhanden sein. In vielen Fällen tritt jedoch Hirnödem, Nierenversagen und schließlich Multiorganversagen hinzu. Abhängig von der Ätiologie können unterschiedliche Symptome bevorzugt sein. Beispielsweise ist bekannt, daß bei Paracetamolvergiftung Enzephalopathie und schwere Koagulopathie mit nur geringem Ikterus auftreten, während eine fulminante Non-A non-B Hepatitis häufig mit einem schweren Ikterus vergesellschaftet ist.

Der zeitliche Faktor erlaubt eine gewisse prognostische Aussage. Paradoxerweise hat die Gruppe von Patienten, bei denen sich die Enzephalopathie am schnellsten entwickelt, die größe Chance auf Spontanremission (Gimson 1986, Benhamou 1991). Somit wird ein hyperakutes, akutes und subakutes Leberversagen unterschieden (O'Grady 1993). Hyperakutes Leberversagen wird benutzt, um Patienten zu beschreiben, bei denen die Enzephalopathie innerhalb von 8 Tagen nach Beginn des Ikterus einsetzt. In Hinblick auf die Ätiologie gehören die Paracetamolvergiftung und manchmal die akute Hepatitis A und B am ehesten in diese Gruppe. Das akute Leberversagen umfaßt Patienten mit einer Zeitspanne von acht bis achtundzwanzig Tagen zwischen Ikterus und Enzephalopathie. Die meisten Leberversagen viraler Ätiologie präsentieren sich derart. Beim subakuten Leberversagen tritt die Enzephalopathie mit einer Latenz von vier bis sechsundzwanzig Wochen auf. Die meisten Patienten dieser Gruppe haben eine sogenannte non-A non-B Hepatitis, wo kein virales Agens identifiziert werden kann.

Über 50 % der Fälle von akutem Leberversagen in nordamerikanischen und europäischen Zentren haben die Verlaufsform und das klinische Bild einer akuten viralen Hepatitis, obwohl in vielen Fällen kein spezifischer viraler Hepatitiserreger identifiziert werden kann (Fagan und Harrison 1994). In den meisten Serien ist Hepatitis B der häufigste virale Hepatitiserreger, der ein akutes Leberversagen verursachen kann, gefolgt von non-A non-B und Hepatitis A. Das gilt besonders in Frankreich, wo 46% aller Patienten mit akutem Leberversagen HBV positiv sind. In Japan sind es sogar 62%. Aufgrund der niedrigen Prevalenz von HBV Infektionen in Großbritannien ist im Vereinigten Königreich die non-A non-B Hepatitis die häufigste virale Ursache eines akuten Leberversagens. Hepatitis C spielt in den großen Serien der westlichen Welt eine untergeordnete Rolle (Sallie et al 1994), obwohl wenige gut dokumentierte Fallberichte von fulminanter Hepatitis C publiziert sind (Theilmann 1992).

Von 342 Fällen von akutem Leberversagen, die von 1993 - 1994 mit Enzephalopathie - Grad III bzw. IV im King's College, London aufgenommen wurden, lag in 250 Fällen eine Paracetamol (Acetaminophen)-Vergiftung vor. Eine virale Genese wurde bei 44 Patienten angenommen (Hepatitis A n=8, Hepatitis B n=8, Non-A,B,C,D,E n=28). Seltene Ursachen waren Morbus Wilson, Schwangerschaftsgestosen, Lymphom/maligne Transformation, Sepsis, Budd-Chiari Syndrom, Ischämische Hepatitis, und Reaktionen auf Medikamente (z.B. Cyproterone, Nichtsteroidale Antiphlogistika, Chloroquin, Rifampicin, Isoniazid, Halothane, Flucloxacillin).

Zum Zeitpunkt der Einlieferung des Patienten ist die maßgebliche Leberschädigung bereits abgelaufen. Die histologische Untersuchung der Leber zeigt zu diesem Zeitpunkt Nekrosezonen und - abhängig von der Latenz - auch bereits Regenerationszonen. Die klinischen Symptome und der Verlauf der Krankheit hängen vom Zusammenspiel dreier Faktoren ab: 1) der Regenerationsfähigkeit der Leber, 2) den adversen metabolischen Konsequenzen eines Leberausfalls und 3) der Abgabe von proinflammatorischen, teils toxischen Mediatoren aus der nekrotischen Leber. Charakteristischerweise zieht ein Leberversagen ein Multiorganversagen nach sich.

Die Prognose wird vom Vorliegen einer Enzephalopathie mit konsekutivem Hirnödem entscheidend beeinflußt. Patienten mit akutem Leberversagen, deren Enzaphalopathie nicht über Grad I-II hinaus fortschreitet, haben eine exzellente Prognose, während die Patientengruppe mit Grad III-IV eine wesentlich höhere Mortalität aufweist. Üblicherweise entwickeln 80% der Patienten mit einer Enzephalopathie Grad IV ein Hirnödem (O'Grady et al. 1988). Die pathogenetischen Faktoren, die zur hepatischen Enzephalopathie und Hirnödem führen, sind noch wenig untersucht. Es kann zwischen vasogenen Faktoren, die aufgrund der gestörten Blut-Hirnschranke zu einem extrazellulären Ödem führen und zytotoxischen Mediatoren, die zum intrazellulären Ödem führen, unterschieden werden. Als letztere kommen Benzodiazepinagonisten, ein veränderter Gamma-Aminobuttersäurestatus, eine erhöhte Konzentration an aromatischen Aminen, Ammoniak und Mercaptane in Betracht. Basile et al. (1991) konnte in Gehirnen von Patienten mit akutem Leberversagen erhöhte Konzentrationen von 1,4-Benzodiazepinen nachweisen.

Die klinischen Zeichen, die aufgrund des erhöhten intracraniellen Drucks resultieren, sind Koma, systemische Hypertension, abnorme Pupillenreflexe und Beeinträchtigung der Hirnstammreflexe. Der erhöhte intracranielle Druck bedingt weiter eine verminderte cerebrale Durchblutung (Almdal et al. 1989, Sari et al. 1990) mit konsekutiver cerebraler Ischämie und manchmal epileptiformer Aktivität.

Eine Hypoglykämie tritt im klinischen Verlauf frühzeitig auf und ist die Folge von vermehrt zirkulierendem Insulin bei gleichzeitig beeinträchtigter Gluconeogenese und vermindertem Glucogenabbau. Zudem wird auch eine Hypophosphatämie frühzeitig beobachtet. Die häufig vorgefundene metabolische Azidose kann durch den eingeschränkten Laktatmetabolismus der Leber und durch Gewebshypoxie mit zunehmender peripherer Laktatbildung (Bihari, 1985) erklärt werden.

Infektionen sind eine häufige Komplikation des akuten Leberversagens, wobei angenommen werden muß, daß im Leberversagen vor allem die Funktion der neutrophilen Leukozyten und der Kupfferschen Sternzellen sowie die Opsonierung beeinträchtigt ist.

Dies führt zu einer beeinträchtigten Clearance von Endotoxinen und einer Translokation von Bakterien durch die Mucosabarriere des Darms.

In der Serie vom King's College wurden in 80% der Patienten mit akutem Leberversagen bakterielle Infektionen identifiziert, wobei kulturell Staphylokokkus aureus als häufigster Erreger nachgewiesen wurde (Rolando et al. 1990). Pilzinfektionen, meist Candida albicans, gewinnen im späteren Verlauf der Erkrankung zunehmend Bedeutung (Rolando 1991).

Ein weiteres charakteristisches Leitsymptom des akuten Leberversagens ist die Koagulopathie. Die Prothrombinzeit korreliert gut mit dem Schweregrad des Leberschadens. Da der Gerinnungsfaktor V die kürzeste Halbwertszeit aufweist, ist er der sensitivste Parameter für die Koagulopathie. Diese ist allerdings nicht nur durch eine eingeschränkte Synthese von Gerinnungsfaktoren erklärbar. Zusätzlich tritt noch im Rahmen einer disseminierten intravasalen Gerinnung (DIG) ein vermehrter peripherer Sauerstoffverbrauch auf (O'Grady et al. 1986). Auch die Plättchenfunktion ist eingeschränkt; Thrombozytopenien und eingeschränkte Aggregation bei akutem Leberversagen sind beschrieben worden.

Die orthotope Lebertransplantation ist Therapie der Wahl beim fulminanten und chronischen Leberversagen. Wir werden jedoch zunehmend mit einem eklatanten Organmangel konfrontiert. Daten aus den USA zeigen, daß alle 30 Minuten ein Patient für eine Transplantation gelistet, während nur etwa alle 2 Stunden ein Spender gemeldet wird. Ähnlich ist die Situation auch hierzulande. Im Jahr 1996 erfolgten im Eurotransplant-Raum 973 Lebertransplantationen, während im gleichen Zeitraum 1393 neue Patienten auf der Warteliste für eine Lebertransplantation registriert wurden. 200 (12%) Patienten sind im Jahr 1996 auf der Warteliste für eine Lebertransplantation verstorben.

Eine Lebertransplantation ist beim akuten Leberversagen oft unumgänglich, weil sich nach fulminantem Verlauf infektiöser Hepatitiden oder Intoxikation (Knollenblätter-Pilz, Acetaminophen, Tetrachlorkohlenstoff, etc.) die Leber nicht schnell genug regeneriert. Die Mortalität ohne Transplantation wird in der Literatur zwischen 70%-90% angegeben. Wenn auch von mehreren Gruppen klinische Kriterien für die Indikation zur Lebertransplantation formuliert wurden, so kann es äußerst schwierig sein, den richtigen Zeitpunkt der Transplantation festzulegen oder ein geeignetes Organ rechtzeitig zu bekommen. Ein temporärer Leberersatz würde es ermöglichen, daß der Patient die Phase der Leberregeneration ohne die kostspielige und auf Grund des Organmangels oft nicht rechtzeitig verfügbare Transplantation übersteht. Auch könnte dann auf die lebenslange Immunsuppression mit all ihren Nebenwirkungen verzichtet werden. Als temporärer Leberersatz wurde eine Vielzahl unterschiedlicher Verfahren erprobt. Hämodialyse, Hämadsorption mit Aktivkohle, Affinitätschromatographie zur Entfernung von Stoffwechselprodukten und viele andere Techniken haben keine weitverbreitete Akzeptanz gefunden. Die vielversprechendste Entwicklung ist die der "bioartifiziellen" Leber. In Anbetracht der Komplexität der metabolischen und physiologischen Funktionen der Leber enthält dieses extrakorporale Leber-Ersatzsystem lebende Hepatozyten. Da humane Leberzellen nicht in ausreichendem Maß zur Verfügung stehen und in Kultur nur unzulänglich vermehrt werden können, wurden entweder Hepatomzell-Linien oder xenogene Hepatozyten vom Schwein eingesetzt. Dies ist mit entsprechender Sicherheit für den Patienten nur dann möglich, wenn Patientenplasma oder Blut nicht in direkten Kontakt mit dem Leberzell-Kompartment treten. Nur so können unerwünschte Immunreaktionen und Infektionen verhindert werden. Eine semipermeable Membran trennt daher Blut/Plasma auf der einen Seite von den Hepatozyten auf der anderen und ermöglicht dennoch den notwendigen Stoffaustausch. Einzelne Fallberichte über den kontinuierlichen Einsatz einer solchen bioartifiziellen Leber bei Patienten mit fulminantem Leberversagen gaben vorerst Anlaß zur Hoffnung. Allerdings konnten kontrollierte klinische Studien mit diesen teils käuflich erhältlichen Systemen keinen Vorteil hinsichtlich des Überlebens für Patienten mit akutem Leberversagen zeigen.

Beispiele für bekannte Geräte und Systeme zeigen die US-Patente 3,734,851, 5,043,260, 5,605,835, 5,827,729 und 6,008,049. Die beiden letztgenannten zeigen auch ein dreikammeriges Gerät, das als bioartifizielle Leber eingesetzt werden kann.

Die Erfindung hat es sich nun zur Aufgabe gestellt, ein bioartifizielles Gerät der eingangs genannten Art zu schaffen, in dem eine Kultivierung von Zellen auf mikroskopisch kleinen Trägerpartikeln (Microcarriern) und bevorzugt dessen Einsatz als extrakorporales System unter folgenden Bedingungen möglich wird:
1. Ausreichender Platz für die in einer Suspension enthaltenen Zellen (beispielsweise 2 x 10¹⁰ Leberzellen)
2. Maximale Austauschoberfläche
3. Stoffaustausch bis zu einer maximalen Masse von etwa 100 000 bis 120 000 Dalton
4. Ausschluß von Gasblasen und bevorzugt:
5. Einhaltung von Mikrograviditätsbedingungen, dh extrem niedrige Scherkräfte und Turbulenzen
6. Kontinuierliche Zufuhr von sauerstoffgesättigtem Nährmedium.

Ein erfindungsgemäßes Gerät ist nun dadurch gekennzeichnet, daß die Durchflußkammer durch einen schraubenförmig gewickelten Schlauch aus einem semipermeablen Material gebildet ist, der in der Zellkulturkammer angeordnet ist. Diese Anordnung schafft eine maximale Austauschfläche bei einfachster Konstruktion, da die Durchflußkammer praktisch vollständig von der insbesondere in Form einer Suspension vorgesehenen Zellkultur umgeben ist. Im Gegensatz zu Reaktoren, in denen mehrere gerade parallel zueinander angeordnete Durchflußkammern in Form von flachen Schlitzen, Hohlfasern oder dergleichen vorgesehen sind, weist das erfindungsgemäße Gerät den Vorteil auf, daß anstelle mehrerer paralleler Anschlüsse für Zufluß und Abfluß nur eine einzige Anschlußverbindung zwischen dem Schlauch und dem Zufluß bzw. dem Abfluß vorgesehen sein muß, wobei aufgrund der schraubenförmigen Wicklung am einzigen Schlauch die benötigte große Austauschoberfläche gegeben ist.

Um das bioartifizielle Gerät im Sinne einer bioartifiziellen Leber auch zur Reinigung bzw. Regenerierung von Plasma oder Blut heranziehen zu können, sieht eine bevorzugte Ausführung vor, daß zwei Durchflußkammern voneinander getrennt in der Zellkulturkammer angeordnet sind, wobei bevorzugt jede der beiden Durchflußkammern durch einen schraubenförmig gewickelten Schlauch aus einem semipermeablen Material gebildet ist. Beide Schläuche erstrecken sich durch eine Reaktionskammer, in der die auf Microcarriern adhärierenden Zellen in einer Suspension enthalten sind, wobei es sowohl für die Versorgung der Zellen mit dem Nährmedium als auch für den Stoffaustausch mit dem Plasma oder Blut von Vorteil ist, wenn jeder Schlauch einem rotierend angetriebenen Träger zugeordnet ist. Die Zellen werden dadurch in der Suspension in Schwebe gehalten, und aufgrund des Rühreffektes auch gleichmäßiger und besser mit dem Nährmedium versorgt. Da in der Verwendung als bioartifizielle Leber die Austauschfläche zwischen Plasma/Blut und der Zellsuspension wesentlich größer sein sollte, als die Austauschfläche zwischen dem Nährmedium und der Zellsuspension ist weiters bevorzugt vorgesehen, daß der angetriebene Träger für den vom Nährmedium durchflossenen Schlauch mit einer zentralen Welle verbunden ist, sowie daß der angetriebene Träger für den von Plasma oder Blut durchflossenen Schlauch nahe der Außenwand des Reaktorgefäßes angeordnet ist. Die Durchflußrichtungen des Nährmediums und des Plasmas bzw. Blutes sind bevorzugt entgegengesetzt.

Beide Schläuche bestehen bevorzugt aus einem Polyvinylidendifluorid (PVDF) und zumindest der äußere Schlauch hat eine Porengröße entsprechend etwa 100 000 Dalton.

Die Rotationsgeschwindigkeit ist variabel und sollte zwischen 20 und 40 Umdrehungen pro Minute angesetzt werden. Die innere Welle und die äußere Zylinderwand werden bevorzugt getrennt angetrieben, sodaß beispielsweise auch die innere Welle schneller angetrieben werden kann, um einem Gradienten zwischen Außen- und Innenwand entgegenzuwirken. Das Volumen des Zylinderinneren beträgt vorzugsweise etwa ein Liter. Es wird mit einer Suspensionkultur von Hepatozyten gasblasenfrei gefüllt. Die Zellsuspension enthält in diesem Fall etwa 2 x 10¹⁰ Zellen, was etwa der Ausbeute einer Schweineleber entspricht. Die äußere Schlauchwendel für Plasma/Blut umfaßt bevorzugt eine Oberfläche von ca. 4,5 m². Der Bioreaktor wird durch einen Brutschrank oder einen Wassermantel auf 37°C temperiert. Aufgrund der kontinuierlichen Versorgung mit Sauerstoff aus dem mit Sauerstoff gesättigten Nährmedium durch die Wandung des inneren Schlauchs und dem bevorzugt mit Sauerstoff angereicherten Plasma bzw. Blut durch die Wandung des äußeren Schlauchs kann eine durchschnittliche Lebensdauer der Leberzellen von 20 Tagen erreicht werden, wobei eine sehr gute bis ausreichende Funktionalität über mindestens 14 Tage festgestellt worden ist.

Bevorzugt ist das Reaktorgefäß zylindrisch ausgebildet und weist eine horizontal angeordnete Achse auf.

Die Zu- und Ablaufanschlüsse der bzw. jeder Durchflußkammer können in den beiden Stirnseiten des Reaktorgefäßes angeordnet sein. Bevorzugt sind sie an derselben Seite angeordnet, wobei der Schlauch vom Ende der Wendel auf unterschiedliche Weise zurückgeführt sein kann. Eine erste Möglichkeit sieht eine zweite schraubenförmige Wicklung vor, eine zweite Möglichkeit einen geraden Rückflußabschnitt und eine bevorzugte dritte Variante ein starres Rückflußrohr, das insbesondere in der zentralen Welle verlaufen kann.

Alternativ kann das bioartifizielle Gerät stationäre Anschlüsse und feststehende konzentrische Schlauchwendeln aufweisen. In diesem Fall ist das Reaktorgefäß auf einem feststehenden Lagerblock, an dem die Schlauchwendeln befestigt sind, drehbar gelagert und mit einem äußeren Antrieb versehen. Ein schonender Rühreffekt in der Zellkultursuspension kann dabei dadurch erzielt werden, daß das insbesondere topfartige Reaktorgefäß ein oder mehrere zwischen die Schlauchwendeln ragende Flügel od. dgl. aufweist, die vom Gefäßboden hochstehen.

Nachstehend wird nun die Erfindung anhand der Figuren der beiliegenden Zeichnung näher beschrieben, ohne darauf beschränkt zu sein.

Es zeigen:
- Fig. 1: einen Axialschnitt durch eine erste Ausführung eines bioartifiziellen Gerätes mit Leberfunktion nach der Linie I-I der Fig. 3,
- Fig. 2: einen um 90° versetzten Axialschnitt durch das bioartifizielle Gerät nach der Linie II-II der Fig. 3,
- Fig. 3: eine Stirnansicht,
- Fig. 4: ein Säulendiagramm der Überlebenszeiten dreier Versuchstiere,
- Fig. 5: ein zugehöriges Diagramm über den intracraniellen Druckverlauf der Versuchstiere, und
- Fig. 6: eine zweite Ausführung eines bioartifiziellen Gerätes mit Leberfunktion in einem Axialschnitt gemäß Fig. 1.

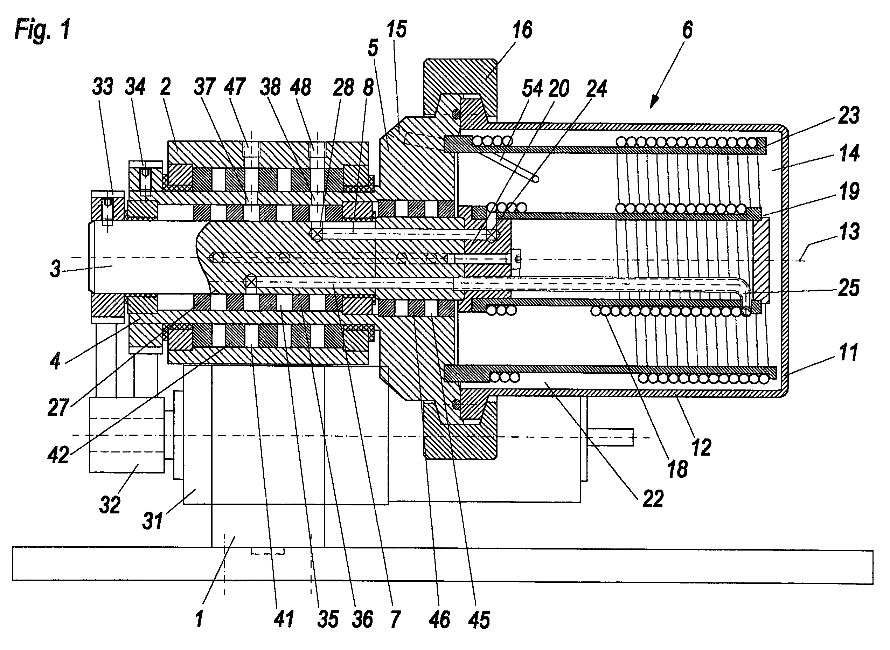

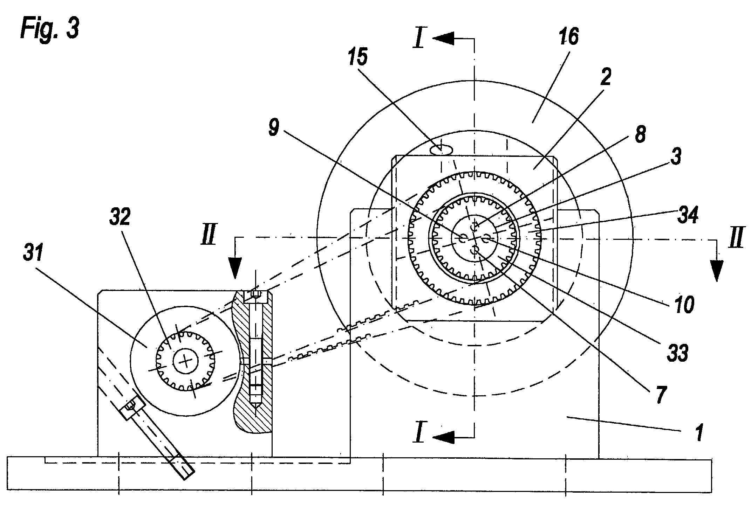

Gemäß Fig. 1 bis 3 weist ein bioartifizielles Gerät ein Reaktorgefäß 6 auf, das um eine horizontale Achse 13 drehbar in einem Grundgestell 1 angeordnet ist. In das Grundgestell 1 ist ein Lagerblock 2 vertikal eingeschoben, in dem eine hohlzylindrische äußere Antriebswelle 4 gelagert ist, die einen endseitigen Flansch 5 aufweist. Der Flansch 5 und ein mit diesem über einen leicht lösbaren Verschlußring 16 verbundener Gehäusetopf 11 aus insbesondere durchsichtigem Material bilden das Reaktorgefäß 6. In der hohlzylindrischen Antriebswelle 4 ist eine zentrale Antriebswelle 3 drehbar gelagert.

Beide Antriebswellen 3, 4 sind mit Antriebsübertragungselementen 33, 34, beispielsweise Zahnrädern, Riemenscheiben od. dgl. versehen, die über ein entsprechendes Zugmittel mit einem Abtriebselement 32 eines Motors 31 verbunden sind. Das Antriebsübertragungselement 34 weist bevorzugt einen größeren Außendurchmesser auf, sodaß die Drehzahl der Antriebswelle 4 kleiner als die Drehzahl der Antriebswelle 3 ist.

Das Reaktorgefäß 6 weist eine Zellkulturkammer 14 auf, die über mindestens eine im Flansch 5 ausgebildete Prüföffnung 15 zugänglich ist. Über diese kann eine Probeentnahme der Zellkultur ebenso erfolgen wie eine Entnahme bzw. Zufuhr, um eventuelle Druckänderungen im Reaktorgefäß 6 auszugleichen. Von der bzw. einer Prüföffnung 15 erstreckt sich vorzugsweise ein Rohr- oder Schlauchstück 54 ins Innere der Zellkulturkammer 14, um eine Probenentnahme aus einem nicht randständigen Bereich zu ermöglichen.

Durch die Zellkulturkammer 14 führt eine Durchflußkammer 17 für ein Nährmedium und eine Durchflußkammer 21 für Plasma bzw. Blut. Die Durchflußkammem 17 und 21 sind voneinander getrennt angeordnet und jeweils durch einen Schlauch 18, 22 gebildet, der aus einem semipermeablen Material, insbesondere einem Polyvinylidendifluorid (PVDF) besteht, dessen Porengröße maximal etwa 100.000 Dalton entspricht. Die Durchflußkammer 17 für das Nährmedium ist durch den Schlauch 18 gebildet, der auf einem Träger 19 schraubenförmig gewickelt ist, der mehrere die Windungen aufnehmende Trägerleisten aufweist. Der Träger 19 steht stirnseitig von einem Verbindungselement 20 ab, das an der zentralen Antriebswelle 3 befestigt ist. Der Träger 19 und der Schlauch 18 drehen sich daher mit der Antriebswelle 3 mit. Der Schlauch 22, der die Durchflußkammer 21 für Plasma bzw. Blut bildet, ist ebenfalls auf zwei bis acht Trägerleisten schraubenförmig aufgewickelt, die den Träger 23 bilden. Die Trägerleisten des Trägers 23 stehen nahe der Umfangswand 12 des Reaktorgefäßes 6 vom Flansch 5 ab, der an der bevorzugt langsamer drehenden äußeren Antriebswelle 4 vorgesehen ist.

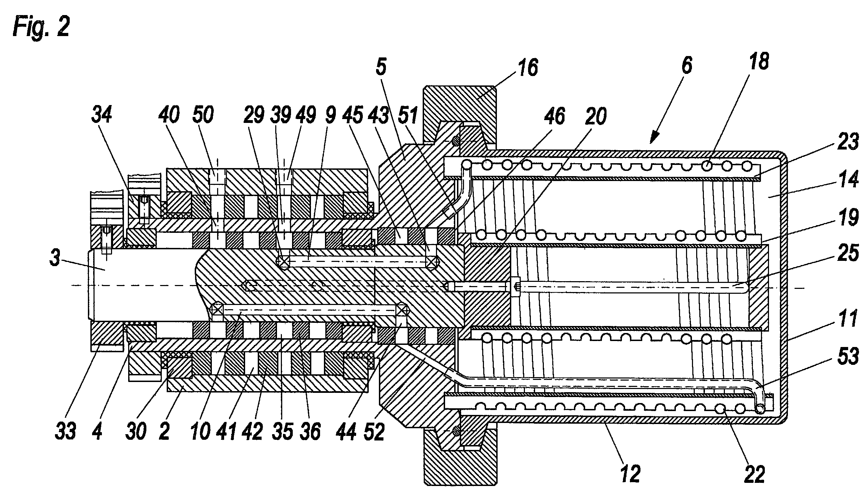

Beide Schläuche 18, 22 tauchen in die Zellkulturkammer 14 ein, wobei durch die horizontale Drehachse 13 und den bevorzugten Drehzahlunterschied zwischen den beiden Schlauchwendeln ein geringfügig schonender Rühreffekt in der Zellkultur gegeben ist, durch den der Stoffaustausch zwischen dem Nährmedium im zentralen Schlauch 18 und der Zellkultur sowie zwischen dem Plasma bzw. Blut im äußeren Schlauch 22 und der Zellkultur begünstigt wird. Die schraubenförmige Anordnung der beiden Schläuche ergibt weiters eine sehr große Austauschfläche zwischen den einzelnen Medien. Die Schlauchwindungen können einander berührend (Fig. 1) oder mit Abstand (Fig. 2) auf den Trägern 19, 23 angeordnet sein. Eine berührende Anordnung ergibt aufgrund einer höheren Anzahl von Windungen eine größere Gesamtlänge, während eine beabstandete Anordnung den Austausch auf dem gesamten Umfang des Schlauches ermöglicht, da die gegenseitige Abdeckung im Berührungsbereich der Windungen entfällt.

Der Zufluß des Nährmediums und des Plasmas bzw. Blutes in die Schläuche 18, 22 und deren Rückfluß erfolgen jeweils über die zentrale Antriebswelle 3 in voneinander getrennten Strömungskanälen. Am Lagerblock 2 sind Anschlüsse 47, 48, 49, 50 vorgesehen, die mit radialen Bohrungen 27, 28, 29, 30 der zentralen Antriebswelle 3 in Strömungsverbindung stehen. Zwischen der Innenwand des Lagerblocks 2 und der äußeren Antriebswelle 4 sind pro Zufluß bzw. Rückfluß äußere Ringkanäle 41 vorgesehen, die durch axial beabstandete Trennringe 42 begrenzt sind und die jeweils Strömungsverbindungen zwischen den Anschlüssen 47, 48, 49, 50 und radialen Bohrungen 37, 38, 39, 40 der sich drehenden äußeren Antriebswelle 4 herstellen. Zwischen der Innenwand der äußeren Antriebswelle 4 und der zentralen Antriebswelle 3 sind pro Zufluß bzw. Rückfluß innere Ringkanäle 35 vorgesehen, die durch axial beabstandete Trennringe 36 begrenzt sind und die jeweilige Strömungsverbindung zwischen den radialen Bohrungen 37, 38, 39, 40 der äußeren Antriebswelle 4 und den Bohrungen 27, 28, 29, 30 der sich schneller drehenden zentralen Antriebswelle 3 herstellen.

Die zentrale Antriebswelle 3 enthält vier zueinander parallele Strömungskanäle 7, 8, 9, 10. Von diesen ist der Strömungskanal 7 durch ein in den Gehäusetopf 11 ragendes Verlängerungsrohr 25 verlängert, dessen radial abgebogenes Ende mit dem dem Flansch 5 gegenüberliegenden, ersten Ende des Schlauches 18 verbunden ist. Der Strömungskanal 8 ist in ein Verbindungselement 20 verlängert, und endet in einer radialen Eintrittsbohrung 24, an die das flanschnahe zweite Ende des Schlauchs 18 angeschlossen ist. Nährmedium zur Versorgung der Zellkultur fließt somit über den ersten Anschluß 47 in den äußeren Ringkanal 41, durch die Bohrung 37 der äußeren Antriebswelle 4 in den inneren Ringkanal 35, durch die Bohrung 27 der zentralen Antriebswelle 3 in deren ersten Strömungskanal 7 und durch das Verlängerungsrohr 25 in den Schlauch 18. Aus dem Schlauch 18 fließt das zirkulierende Nährmedium durch die Eintrittsöffnung 24, den zweiten Strömungskanal 8 und die radiale Bohrung 28 der zentralen Antriebswelle 3 in den zugehörigen inneren Ringkanal 35, durch die Bohrung 38 der äußeren Antriebswelle 4 und den zugehörigen äußeren Ringkanal 41 zum zweiten Anschluß 48 des Lagerblocks 2.

Die Strömungskanäle 9, 10 der zentralen Antriebswelle 3 sind über innere radiale Bohrungen 43, 44 mit Ringkanälen 45 verbunden, die durch Trennringe 46 begrenzt und zwischen der zentralen Antriebwelle 3 und dem Flansch 5 der äußeren Antriebswelle 4 angeordnet sind. Der Flansch 5 weist Kanäle 51, 52 auf, die in den Bereich des äußeren Trägers 23 führen. Am Ausgang des Kanales 51 ist der Schlauch 22 angeschlossen. Vom Ausgang des Kanales 52 führt ein Verlängerungsrohr 53 zum freien Ende des Trägers 23, an dem das zweite Ende des Schlauches 22 mit dem Verlängerungsrohr 53 verbunden ist.

Plasma bzw. Blut strömt über den dritten Anschluß 49 in das bioartifizielle Gerät ein und gelangt durch den zugehörigen äußeren Ringkanal 41 und die Bohrung 39 der äußeren Antriebswelle 4 in den zugehörigen inneren Ringkanal 35, durch die Bohrung 29 der zentralen Antriebswelle 3 in deren dritten Strömungskanal 9, durch die zweite, innere Bohrung 43 am Ende des Strömungskanals 9 in den zugehörigen Ringkanal 45 und durch den Kanal 51 im Flansch 5 zur äußeren Schlauchwendel 22, in der durch Stoffaustausch mit der Zellkultur in der Zellkulturkammer 14 eine Reinigung des Plasmas bzw. Blutes erfolgt. Das behandelte Plasma bzw. Blut fließt durch das Verlängerungsrohr 53 und den Kanal 52 im Flansch 5 in den zugehörigen Ringkanal 45, durch die innere Bohrung 44 der zentralen Antriebswelle 3 in deren vierten Strömungskanal 10, durch die äußere Bohrung 30 der zentralen Antriebswelle 3 in den zugehörigen inneren Ringkanal 35, und durch die Bohrung 40 der äußeren Antriebswelle 4 und den zugehörigen Ringkanal 41 zum vierten Anschluß 50 des Lagerblockes 2.

Für die Erstbefüllung bzw. Entleerung wird der Lagerblock 2 aus dem Grundgestell 1 entnommen, sodaß das Reaktorgefäß 6 aufgestellt werden kann, wobei die Achse 13 lotrecht verläuft. Nach Abnahme des Verschlußringes 16 kann der Lagerblock 2 mit dem Flansch 5 und den auf den beiden Trägern 19, 23 angeordneten Schlauchwendeln 18, 22 nach oben abgenommen werden. Die verbrauchte Zellkultur kann entleert bzw. neue Zellkultur in den Gehäusetopf 11 eingefüllt werden. Die Einheit aus Flansch 5 samit den beiden Schlauchwendeln 18, 22 und dem Lagerblock 2 wird auf den Gehäusetopf 11 aufgesetzt und mit Hilfe des Verschlußringes 16 zum Reaktorgefäß 6 dichtend verschlossen. Anschließend kann das Reaktorgefäß 6 umgelegt und mit Hilfe des Lagerblockes 2 liegend in das Grundgestell 1 eingesteckt werden.

In bevorzugter Ausführung hat der Gehäusetopf 11 einen Rauminhalt von etwa einem Kubikdezimeter und kann ca. 2 x 10¹⁰ in einem Medium suspendierte Hepatozyten aufnehmen. Die Drehzahlen der beiden Antriebszellen 3, 4 liegen insbesondere zwischen 20 und 40 Umdrehungen pro Minute, wobei die Drehzahl der zentralen Antriebswelle 3 beispielsweise dem Eineinviertelfachen der Drehzahl der äußeren Antriebswelle 4 entspricht. In diesem Fall liegen somit die Drehzahlen von 20 und 30 Umdrehungen bis 25 und 37,5 Umdrehungen pro Minute.

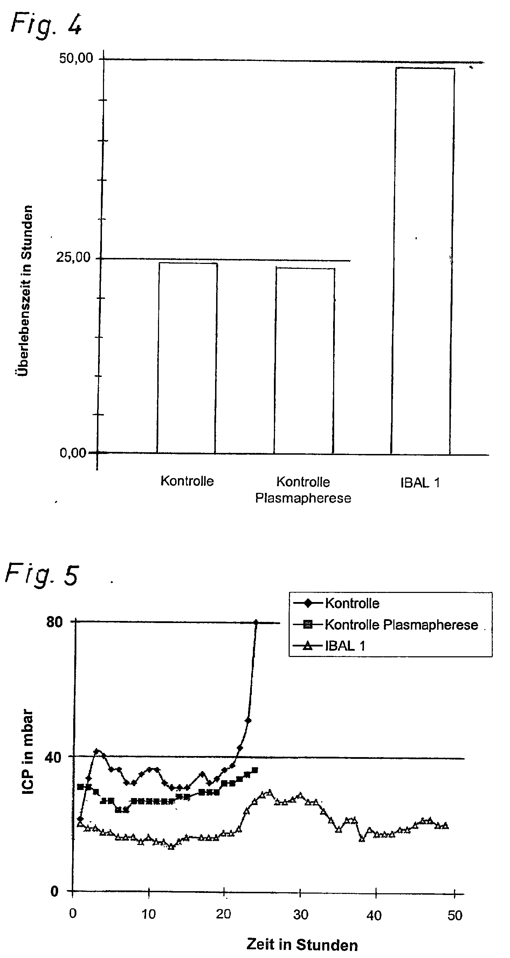

Die Fig. 4 und 5 zeigen das Ergebnis eines Tierversuches, in dem bei drei deutschen Landschweinen mit je etwa 30 kg Lebendgewicht die Überlebensdauer in einem chirurgisch herbeigeführten Modell des akuten Leberversagens ermittelt wurde. Das akute Leberversagen wurde durch eine 80%-Leberresektion mit anschließender einstündiger Abklemmung des Hilus der Restleber herbeigeführt. Weiters wurde unter anderem der intracranielle Druck (ICP) kontrolliert. Da eine Plasmapherese auch zur Therapie bei akuter Leberinsuffizienz eingesetzt wird, wurde folgende Vorgabe getroffen: Eines der drei Tiere blieb ohne Therapie (Kontrolle), bei einem zweiten Tier wurde aus dem Blut in einem Plasmapheresegerät Plasma abgetrennt und als Vollblut ohne weitere Manipulation wieder reinfundiert (Kontrolle Plasmapherese). Beim dritten Tier wurde ebenfalls aus dem Blut in einem Plasmapheresegerät Plasma abgetrennt, und dieses dann durch ein bioartifizielles Gerät mit den oben beschriebenen Angaben hindurchgeführt. Das behandelte Plasma wurde wiederum mit dem zuvor abgetrennten Restblut vermischt und zurückgeführt (IBAL 1).

Fig. 4 zeigt, daß das Kontrolltier und das Plasmapherese-Kontrolltier eine Überlebenszeit von jeweils nahezu 24 Stunden aufwiesen. Hingegen lebte das dritte Tier, dessen Plasma im erfindungsgemäßen bioartifiziellen Gerät gereinigt bzw. regeneriert wurde, nahezu 50 Stunden, wobei die Todesursache, wie Fig. 5 zeigt, nicht in einem Anstieg des intracraniellen Drucks (ICP) gelegen hat. Hingegen stieg der intracranielle Druck beim Kontrolltier extrem, und beim Plasmapherese-Kontrolltier zwar vermindert, aber doch deutlich.

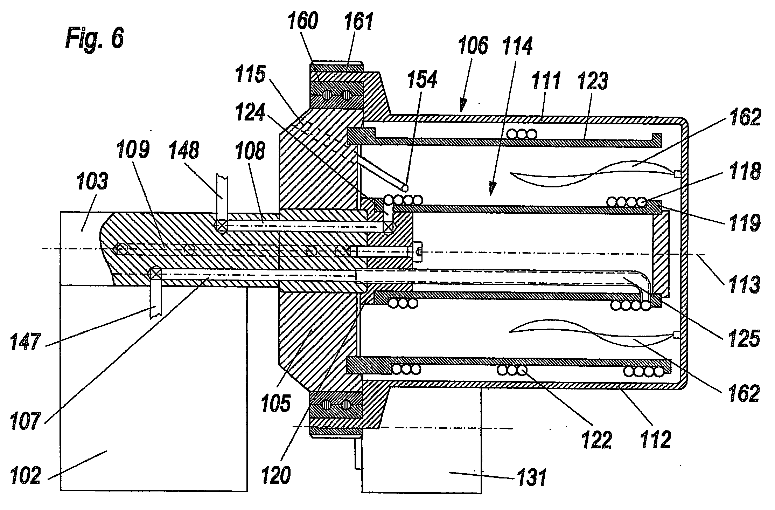

In der Ausführung nach Fig. 6 ist in einen Lagerblock 102 ein zentrales zylindrisches Halteelement 103 abnehmbar angeordnet. Das Halteelement weist die gezeigten Anschlüsse 147, 148, über die Nährmedium zu- und abgeführt werden kann, sowie zwei weitere Anschlüsse auf, über die Blut und Plasma zu- und abgeführt werden kann. Die Anschlüsse 147, 148 münden in Strömungskanäle 107, 108, die sich parallel zur Achse 113 erstrecken. Auf dem Halteelement 103 ist ein Flansch 105 mit einem umfänglichen Wälzlager 160 montiert, auf dem ein Gehäusetopf 111 eines Reaktorgefäßes 106 drehbar gelagert ist. Der Gehäusetopf 111 ist an der Außenseite der Wandung 112 mit einem Zahnkranz 161 versehen, und über ein Antriebsübertragungselement mit einem Motor 131 drehbar verbunden. Der Flansch 105 und der Gehäusetopf 111 des Reaktorgefäßes 106 beinhalten eine Zellkulturkammer 114, in die vom Boden des Gehäusetopfes 111 zwei Rührelemente 162 ragen, die die durch die Rotation der Gehäusewandung 112 bedingte, schonende Durchmischung des Inhalts der Zellkulturkammer 114 unterstützen.

Der Strömungskanal 108 endet in einem Verbindungselement 120, das eine radiale Bohrung 124 aufweist. Der Strömungskanal 107 ist jenseits des Verbindungselementes 120 durch ein Verlängerungsrohr 125 weitergeführt. Ein auf Leisten eines Trägers 119 schraubenförmig gewickelter Schlauch 118 ist einerseits an das Verlängerungsrohr 125 und andererseits an die radiale Bohrung 124 angeschlossen, wobei die Schlauchwendel zentral in Verlängerung des Halteelementes 103 in den rotierenden Gehäusetopf 111 ragt.

Als Durchflußkammer für Blut und Plasma dient ein zweiter auf Leisten eines Trägers 123 schraubenförmig gewickelter Schlauch 122, der über nicht gezeigte im Flansch 105 verlaufende Kanäle mit Strömungskanälen im Halteelement 103 verbunden ist, von denen der Strömungskanal 109 strichliert angedeutet ist. Das Reaktorgefäß 106 weist eine Prüföffnung 115 auf, die über ein inneres Schlauch- oder Rohrstück 154 mit der Zellkulturkammer 114 in Verbindung ist.

## Patentansprüche

1. Bioartifizielles Gerät mit einem Reaktorgefäß (6), in dem eine Zellkulturkammer (14) und eine Durchflußkammer (17) für ein Nährmedium durch eine semipermeable Wandung getrennt sind, **dadurch gekennzeichnet, daß** die Durchflußkammer (17, 21) durch einen schraubenförmig gewickelten Schlauch (18) aus einem semipermeablen Material gebildet ist, der in der Zellkulturkammer (14) angeordnet ist.

2. Bioartifizielles Gerät, insbesondere mit Leberfunktion, mit einem Reaktorgefäß (6), in dem eine Zellkulturkammer (14) durch semipermeable Wandungen einerseits von einer Durchflußkammer (17) für ein Nährmedium und andererseits von einer Durchflußkammer (21) für Plasma oder Blut abgeteilt ist, **dadurch gekennzeichnet, daß** beide Durchflußkammern (17, 21) jeweils durch einen schraubenförmig gewickelten Schlauch (18, 22) aus einem semipermeablen Material gebildet sind, und beide Schläuche (18, 22) in der Zellkulturkammer (14) angeordnet sind.

3. Gerät nach Anspruch 1 oder 2, **dadurch gekennzeichnet, daß** zumindest ein Schlauch (18,22) auf einem angetriebenen Träger (19, 23) angeordnet ist.

4. Gerät nach Anspruch 3, **dadurch gekennzeichnet, daß** der Träger (19) für den vom Nährmedium durchflossenen Schlauch (18) mit einer zentralen Antriebswelle (3) verbunden ist.

5. Gerät nach Anspruch 3, **dadurch gekennzeichnet, daß** der Träger (23) für den von Plasma oder Blut durchflossenen Schlauch (22) nahe der Außenwand (12) des Reaktorgefäßes (6) angeordnet ist.

6. Gerät nach Anspruch 5, **dadurch gekennzeichnet, daß** der Träger (23) für den von Plasma oder Blut durchflossenen Schlauch (22) mit einem angetriebenen Flansch (5) des Reaktorgefäßes (6) verbunden ist, der auf der zentralen Antriebswelle (3) drehbar gelagert ist.

7. Gerät nach Anspruch 3 und 6, **dadurch gekennzeichnet, daß** die Drehzahlen der beiden angetriebenen Träger (19, 23) unterschiedlich sind.

8. Gerät nach Anspruch 1 oder 2, **dadurch gekennzeichnet, daß** das Reaktorgefäß (106) eine drehbar angeordnete Umfangswand (112) aufweist, und jeder Schlauch (118, 122) auf einem feststehenden Träger (119, 123) angeordnet ist.

9. Gerät nach Anspruch 8, **dadurch gekennzeichnet, daß** der angetriebenen Umfangswand (112) zumindest ein zwischen die Schlauchwendeln ragendes Rührelement (162) zugeordnet ist.

10. Gerät nach einem der Ansprüche 1 bis 9, **dadurch gekennzeichnet, daß** das Reaktorgefäß (1) zylindrisch ausgebildet und dessen Achse (13) horizontal angeordnet ist.

11. Gerät nach einem der Ansprüche 1 bis 10, **dadurch gekennzeichnet, daß** die Zu- und Ablaufanschlüsse (47, 48, 49, 50) der Durchflußkammer (17, 21) an derselben Seite des Reaktorgefäßes (6) angeordnet sind, und ein Ende jedes gewickelten Schlauches (18, 22) an ein durch das Reaktorgefäß (6) geführtes Rohr (25, 53) angeschlossen ist.

12. Gerät nach Anspruch 9 und 11, **dadurch gekennzeichnet, daß** das Reaktorgefäß (106) einen angetriebenen Gehäusetopf (111) aufweist, von dessen Boden das Rührelement (162) hochsteht.

13. Gerät nach einem der Ansprüche 1 bis 12, **dadurch gekennzeichnet, daß** der Zellkulturkammer (14) mindestens eine Prüf- bzw. Druckausgleichsöffnung (15) zugeordnet ist.
